# EUROPEAN PATENT SPECIFICATION

(11) **EP 4 427 674 B1**
(45) Date of publication and mention of the grant of the patent: **15.04.2026**
(21) Application number: 24161121.9
(22) Date of filing: 04.03.2024
(51) Int. Cl.: A61B 5/145

(54) **SUBSTRATE SEALING CONFIGURATIONS AND METHODS**
SUBSTRATVERSIEGELUNGSKONFIGURATIONEN UND -VERFAHREN
CONFIGURATIONS ET PROCÉDÉS DE SCELLEMENT DE SUBSTRAT

(30) Priority: 08.03.2023 US 202363450934 P; 01.02.2024 US 202418429804
(43) Date of publication of application: 11.09.2024
(73) Proprietor: Medtronic MiniMed, Inc., Northridge, CA 91325 (US)
(72) Inventor: Larson, Eric A., Northridge, CA 91325 (US); Thompson, Avery, Northridge, CA 91325 (US); Kaypaghian, Kristina U., Northridge, CA 91325 (US); Ruelas, Jose, Northridge, CA 91325 (US)
(74) Representative: Zimmermann & Partner Patentanwälte mbB

(56) References cited:
- US-A1- 2017 290 533
- US-A1- 2021 030 320
- US-A1- 2021 204 841
- US-A1- 2021 378 560

## Description

### CROSS-REFERENCE TO RELATED APPLICATIONS

This application claims the benefit of and priority to U.S. Provisional Patent Application Serial No. 63/450,934 filed March 8, 2023.

### BACKGROUND

Various types of modern electronic devices, including but not limited to certain medical devices and certain other sensor devices, include or employ one or more electronics substrates that pass through a seal barrier between a sealed environment and an exposed environment, or between two sealed environments, or between two different exposed environments.

For example, certain medical devices employ or include one or more thin film sensor electronics substrates that are configured to be inserted, transcutaneous, through a patient's skin into an exposed environment that contains or may contain a biological analyte. In such devices, it may be desirable to have a section of the thin film substrate extend through one or more seal barriers between the exposed environment and a sealed environment that may contain, for example, one or more electronic circuits, electrical power sources or other components that are to be sealed from the exposed environment.

While aspects of the present disclosure are applicable to many types of devices that include one or more electronics substrates, various examples are described herein with regard to a medical sensor device and, in particular, a medical sensor device with a transcutaneous sensor substrate probe. Certain diseases or conditions may be sensed or monitored, according to modern medical techniques, with sensor devices that include one or more sensor substrate probes configured to be inserted, transcutaneous, into a patient. In some examples described herein, a sensor device with a sensor substrate probe may be configured to monitor glucose levels or concentration in blood or interstitial fluid. However, those skilled in the art will understand that aspects of the present disclosure also apply to other types of sensor devices, other medical devices or other devices configured for non-medical purposes.

Glucose sensors have been developed for use in obtaining an indication of blood glucose levels in a diabetic patient. Such readings can be especially useful in monitoring and/or adjusting a treatment regimen that typically includes the regular administration of insulin to the patient. Blood glucose sensors can be particularly useful in improving medical therapies when used with automated or semi-automated medication infusion pumps.

For the sake of brevity, conventional aspects and technology related to glucose sensors and glucose sensor fabrication may not be described in detail here. However, known and/or conventional aspects of glucose sensors and their manufacturing may include, but are not limited to the type described in: U.S. Pat. Nos. 6,892,085, 7,468,033 and 9,295,786. Glucose or other analyte sensors have been included in or used with infusion devices configured to deliver medication to a patient, such as generally described in U.S. Pat. Nos. 6,554,798 and 6,551,276. In addition, for the sake of brevity, conventional aspects and technology related to sensor inserters may not be described in detail here. However, known and/or conventional aspects of sensor inserters may include, but are not limited to the type described in: U.S. Pat. No. 10,413,183 and United States patent application number 2021/0378560.

US 2017/0290533 A1 relates to an insertion device. US 2021/0378560 A1 relates to a physiological characteristic sensor system.

### SUMMARY

One or more examples and aspects described herein relate to a substrate that passes through a seal barrier between a sealed environment and an exposed environment, or between two (or more) sealed environments.

Particular examples relate to a sensor device that includes a housing configured to be secured to a patient, a seal structure located within the housing, and a sensor probe. The sensor probe has at least one substrate supported by the housing. The substrate has a first length portion and a second length portion, the first length portion being external to the housing and configured to be inserted into the patient when the housing is secured to the patient, the second length portion being in or on the housing and having a section that extends through the seal structure. The section of the second length portion of the at least one substrate that extends through the seal structure has at least one side edge having a tortuous edge pattern that enhances a seal effect of the seal structure at the section of the second length portion of the at least one substrate.

In further examples, the section of the second length portion of the sensor substrate that extends through the seal structure has two side edges, each having a tortuous edge pattern.

In further examples, the undulating tortuous edge pattern includes a wave pattern having a plurality of peaks or a plurality of valleys on each of two side edges.

In further examples, the undulating tortuous edge pattern on at least one side edge includes a wave pattern having a plurality of peaks and a plurality of valleys.

In further examples, the seal structure includes first and second O-rings, and the section of the second length portion of the sensor substrate extends between the first and the second O-rings.

In further examples, the housing includes a base and a cover, and the sensor device includes a stacked arrangement having the first O-ring arranged on the base, the section of the second length portion of the sensor substrate arranged on the first O-ring, the second O-ring arranged on the section of the second length portion of the sensor substrate, and the cover arranged on the second O-ring.

In further examples, the housing includes a base and a cover, wherein the base has a first annular cavity in which the first O-ring resides, and the cover has a second annular cavity in which the second O-ring resides.

In further examples, at least one of the first and second annular cavities has a cross-section shape that tapers from a narrower dimension toward a depth of the cavity, to a wider dimension toward an open side of the cavity to enhance compression of the O-ring residing therein, when the base and the cover are coupled together.

In further examples, each of the first and second annular cavities has a cross-section shape that tapers from a narrower dimension toward a depth of the cavity, to a wider dimension toward an open side of the cavity to enhance compression of the O-rings, when the base and the cover are coupled together.

In further examples, the at least one substrate of the sensor probe includes a first substrate having one or more electrical conductors or electrical components.

In further examples, the at least one substrate of the sensor probe includes a second substrate having one side provided with a coating of a drug or treatment substance, the one side of the second substrate facing away from the first substrate of the sensor probe.

In further examples, the second substrate has a mark or other indicia that identifies the one side provided with the coating of the drug or treatment substance.

In further examples, the second substrate has a second side that faces the first substrate of the sensor probe, and the second side of the second substrate is uncoated.

Further examples relate to an electronics substrate including a body of electrically insulating material having one or more electrically conductive members or electrical components supported thereon. The body has a length portion having a section configured to extend through a seal structure. A section of the length portion of the body of electrically insulating material has at least one side edge having an undulating edge pattern that enhances a seal effect when the section of the length portion extends through the seal structure.

In further examples of that electronic substrate, the undulating edge pattern on at least one side edge comprises a wave pattern having a plurality of peaks or a plurality of valleys.

In further examples of that electronic substrate, the body of electrically insulating material comprises a thin film substrate of a sensor probe.

Further examples relate to a method of making a sensor device, including providing a housing configured to be secured to a patient; arranging a seal structure within the housing; and supporting a sensor probe having at least one substrate by the housing. The substrate has a first length portion and a second length portion. The first length portion is external to the housing and configured to be inserted into the patient when the housing is secured to the patient. The second length portion is in or on the housing and having a section that extends through the seal structure. The section of the second length portion of the at least one substrate that extends through the seal structure has at least one side edge having an undulating edge pattern that enhances a seal effect of the seal structure at the section of the second length portion of the at least one substrate.

In further examples of that method, the undulating edge pattern on the at least one side edge includes a wave pattern having a plurality of peaks or a plurality of valleys.

In further examples of that method, arranging a seal structure within the housing includes: arranging a first O-ring on a base of the housing, the first O-ring having a central opening; arranging a portion of the at least one substrate to extend through the opening in the first O-ring; arranging the second O-ring on the first O-ring, with a central opening of the second O-ring in alignment with the central opening first O-ring; and extending a section of a second portion of the at least one substrate between the first O-ring and the second O-ring.

In further examples of that method, the housing includes a base and a cover configured to couple together; arranging a first O-ring includes locating the first O-ring in a first annular cavity in the base; and arranging the second O-ring includes locating the second O-ring in a second annular cavity in the cover. At least one of the first and second annular cavities has a cross-section shape that tapers from a narrower dimension toward a depth of the cavity, to a wider dimension toward an open side of the cavity to enhance compression of the O-ring residing therein, when the base and the cover are coupled together.

Further disclosed herein is a sensor device, wherein the sensor device includes a housing configured to be secured to a patient and a seal structure located within the housing. A sensor probe having at least one substrate is supported by the housing. The substrate has a first length portion and a second length portion. The first length portion is external to the housing and is configured to be inserted into the patient when the housing is secured to the patient. The second length portion is in or on the housing and has a section that extends through the seal structure. That section has at least one side edge having an undulating edge pattern that enhances a seal effect of the seal structure at the section of the second length portion of the at least one substrate. The undulating edge pattern may be an edge wave pattern having a plurality of peaks or a plurality of valleys.

### BRIEF DESCRIPTION OF THE DRAWINGS

The above and other aspects and features of the present invention will become more apparent to those skilled in the art from the following detailed description of the example embodiments with reference to the accompanying drawings, in which:
FIG. 1 is a side view of an example of a sensor device in an operational state.
FIG. 2 is a cross-section, perspective view of the sensor device in FIG. 1.
FIG. 3 is a partially exploded view of the sensor device in FIG. 1.
FIG. 4 is an enlarged view of a portion of the sensor device in FIG. 2.
FIG. 5 is a top-down view of a portion of the sensor device in FIG. 4.
FIG. 6 is a top-down view of an example of sensor substrates of a sensor probe.
FIG. 7 is a top down view of another example of a sensor substrate of a sensor probe.

### DETAILED DESCRIPTION

Hereinafter, example embodiments will be described in more detail with reference to the accompanying drawings. The present invention, however, may be embodied in various different forms, and should not be construed as being limited to only the illustrated embodiments herein. Rather, these embodiments are provided as examples so that this disclosure will be thorough and complete, and will fully convey the aspects and features of the present invention to those skilled in the art. Accordingly, processes, elements, and techniques that are not necessary to those having ordinary skill in the art for a complete understanding of the aspects and features of the present invention may not be described. Unless otherwise noted, like reference numerals denote like elements throughout the attached drawings and the written description, and thus, descriptions thereof may not be repeated. Further, features or aspects within each example embodiment should typically be considered as available for other similar features or aspects in other example embodiments.

Certain terminology may be used in the following description for the purpose of reference only, and thus are not intended to be limiting. For example, terms such as "top", "bottom", "upper", "lower", "above", and "below" could be used to refer to directions in the drawings to which reference is made. Terms such as "front", "back", "rear", "side", "outboard", and "inboard" could be used to describe the orientation and/or location of portions of the component within a consistent but arbitrary frame of reference which is made clear by reference to the text and the associated drawings describing the component under discussion. Such terminology may include the words specifically mentioned above, derivatives thereof, and words of similar import. Similarly, the terms "first", "second", and other such numerical terms referring to structures do not imply a sequence or order unless clearly indicated by the context.

It will be understood that when an element or feature is referred to as being "on," "connected to," or "coupled to" another element or layer, it can be directly on, connected to, or coupled to the other element or feature, or one or more intervening elements or features may be present. In addition, it will also be understood that when an element or features is referred to as being "between" two elements or features, it can be the only element or feature between the two elements or features, or one or more intervening elements or features may also be present.

The terminology used herein is for the purpose of describing particular embodiments and is not intended to be limiting of the present invention. As used herein, the singular forms "a" and "an" are intended to include the plural forms as well, unless the context clearly indicates otherwise. It will be further understood that the terms "comprises," "comprising," "includes," and "including," "has, " "have, " and "having," when used in this specification, specify the presence of the stated features, integers, steps, operations, elements, and/or components, but do not preclude the presence or addition of one or more other features, integers, steps, operations, elements, components, and/or groups thereof. As used herein, the term "and/or" includes any and all combinations of one or more of the associated listed items. Expressions such as "at least one of," when preceding a list of elements, modify the entire list of elements and do not modify the individual elements of the list.

As used herein, the term "substantially," "about," and similar terms are used as terms of approximation and not as terms of degree, and are intended to account for the inherent variations in measured or calculated values that would be recognized by those of ordinary skill in the art. Further, the use of "may" when describing embodiments of the present invention refers to "one or more embodiments of the present invention." As used herein, the terms "use," "using," and "used" may be considered synonymous with the terms "utilize," "utilizing," and "utilized," respectively.

Unless otherwise defined, all terms (including technical and scientific terms) used herein have the same meaning as commonly understood by one of ordinary skill in the art to which the present invention belongs. It will be further understood that terms, such as those defined in commonly used dictionaries, should be interpreted as having a meaning that is consistent with their meaning in the context of the relevant art and/or the present specification, and should not be interpreted in an idealized or overly formal sense, unless expressly so defined herein.

Various types of modern medical devices, sensor devices or other electronic devices include or employ one or more electronics substrates. In some contexts, the electronics substrates in those devices extend through a seal barrier between a sealed environment and an exposed environment, or between two sealed environments, or between two different exposed environments. It can be desirable to provide a reliable seal in the seal barrier, to inhibit leakage of fluid or vaporized contaminants through the seal barrier over the intended useful life of the device. Accordingly, examples described herein relate to an electronics substrate that passes through a seal barrier, and further examples relate to devices and systems that include one or more of such electronics substrates, where the device or substrate (or both) include one or more features as described herein that enhance the fluid seal of the seal barrier, improve manufacturability and throughput of the device, reduce cost of the device, or any combination thereof.

In particular examples, the electronics substrate includes a thin film, sheet, plate, strip, disc or other shaped member of electrically non-conductive material that contains or supports one or more electrically conductive members or components such as, but not limited to electrical leads, electrical traces, electrical contacts, electronic devices or any combination thereof. The electronically conductive members or components may form or be part of an electronic device or system such as, but not limited to an electronic sensor, an electronic medical device, a capillary sampling device, an infusion tube device, or the like.

The substrate may be configured to be part of, or to operate in a device or system, where at least one first portion of the substrate is arranged in an exposed environment, and at least one second portion of the substrate is arranged in an enclosed or sealed environment. The exposed environment may be, but is not limited to, an environment that is exposed to biological material or exposed to other environmental conditions. The enclosed or sealed environment is sealed from the biological material or environmental conditions and, in some examples, may contain electronics or other components of the device.

Accordingly, the substrate may be configured to pass through one or more sealing members that form a seal barrier between the exposed environment and the enclosed or sealed environment. The seal barrier may be configured to inhibit the passage of biological material or environmental material into the enclosed or sealed environment. Alternatively or in addition, the seal barrier may be configured to inhibit the passage of fluid from the enclosed or sealed environment to the exposed environment. As described herein, the substrate, the seal, or the device (or any combination thereof) may be configured to enhance sealing effects and provide improved sealing across the seal barrier.

Electronics substrates and seals may be employed with or provided in various types of devices or systems including, but not limited to those referenced herein. For purposes of explanation, certain examples described herein relate to a medical sensor device or a biological sensor device such as, but not limited to a blood glucose sensor. In other examples, a medical sensor device or biological sensor device is configured to sense other analytes. In yet other examples, aspects of the sealed substrate configuration described herein may be employed in other types of sensors, medical devices or other devices having electronics on a substrate.

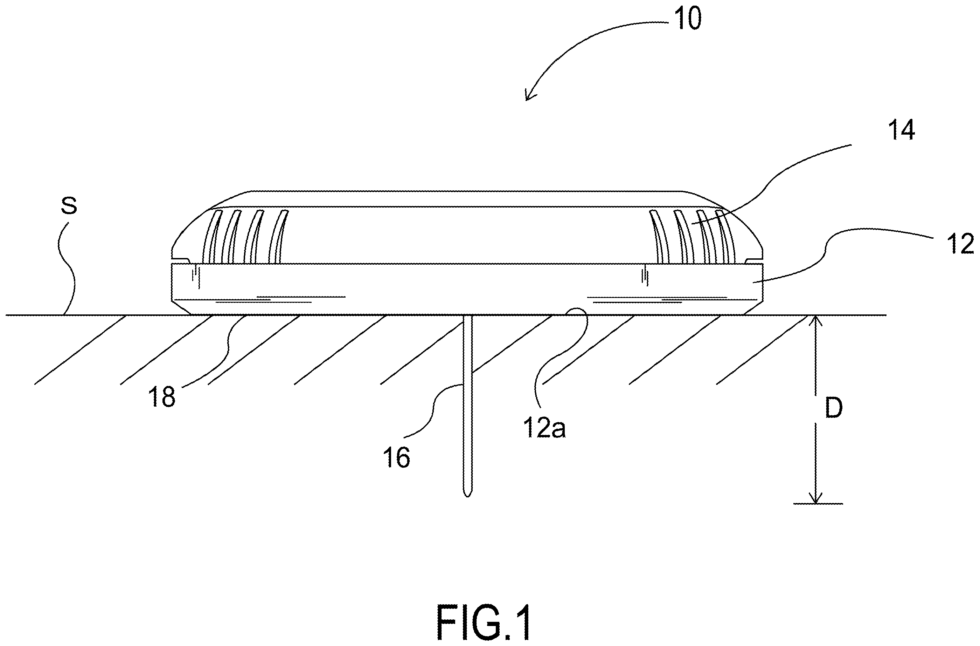

A medical sensor device 10 according to one example is shown in FIGS. 1-3. In the illustrated examples, the sensor device 10 has a generally flat, disc-like or plate-like shape, with rounded edges to avoid snagging on a patient's clothing. However, in other examples, the sensor device 10 may have other suitable shapes.

The sensor device 10 is configured to be coupled to a patient and operate to sense an analyte associated with the patient. The sensor device 10 may be coupled to the patient by pressing the sensor device 10 to the patient's skin S and securing the device 10 to the skin. In certain examples, an insertion needle and an inserter device may be employed to couple the sensor device 10 to the patient. Examples of inserter devices having insertion needles are referenced above. Other examples may employ other suitable inserter devices.

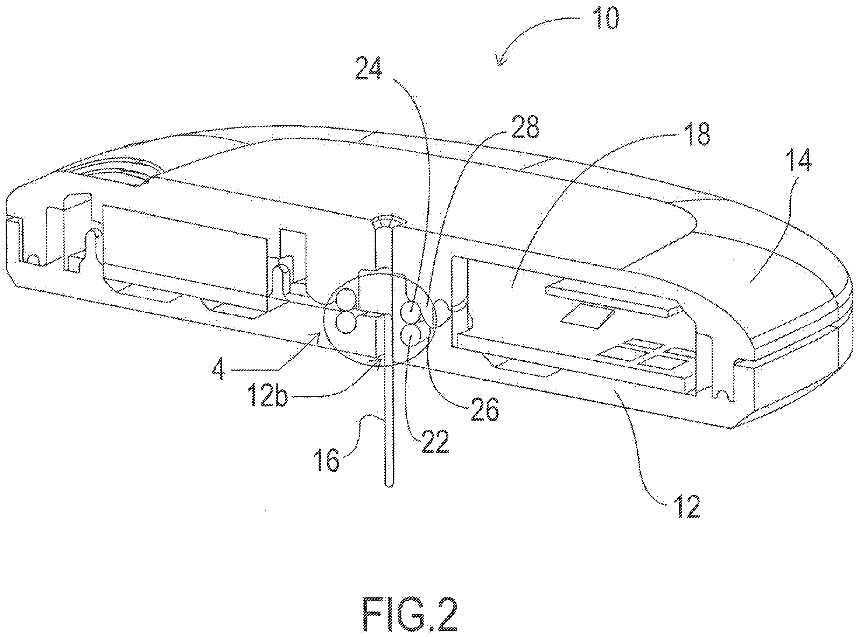

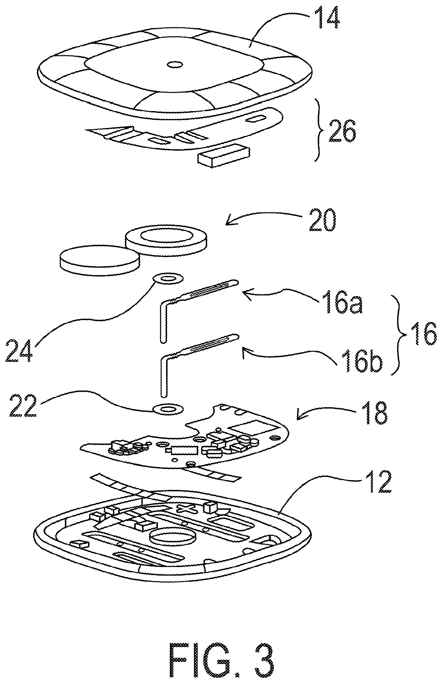

The drawing in FIG. 1 shows a side view of the sensor device 10 in an operational state, after it has been inserted and coupled to a patient's skin S. In that state, the sensor device 10 may be operated to sense or monitor a biological analyte such as, but not limited to a blood glucose level or concentration of the patient. In other examples, the sensor device 10 may be configured to sense or monitor other analytes such as, but not limited to lactate. The sensor device 10 is shown in cross-section view in FIG. 2 and in an exploded view in FIG. 3.

The sensor device 10 includes a base 12, a cover 14 and a sensor probe 16. The base 12 and cover 14 form a housing for holding or containing certain components of the sensor device 10 including, but not limited to a second portion of the sensor probe 16, one or more electronic circuits 18 and one or more batteries 20 or other power sources.

As described in further detail, below, the sensor probe 16 includes one or more substrates that extend through a sealed barrier in the housing formed by the base 12 and the cover 14. In some examples, one or more (or each) substrate may include an electronics substrate. In other examples, one or more (or each) substrate may include a flat capillary tube or other structure. In yet other examples, one or more (or each) substrate may include a combination of an electronics substrate and a flat capillary tube or other structure. A first portion of the sensor probe 16 extends, transcutaneous, into the patient, when the sensor device 10 is coupled to the patient's skin. The first portion of the sensor probe 16 is shown as extending in a generally vertical orientation in FIG. 1.

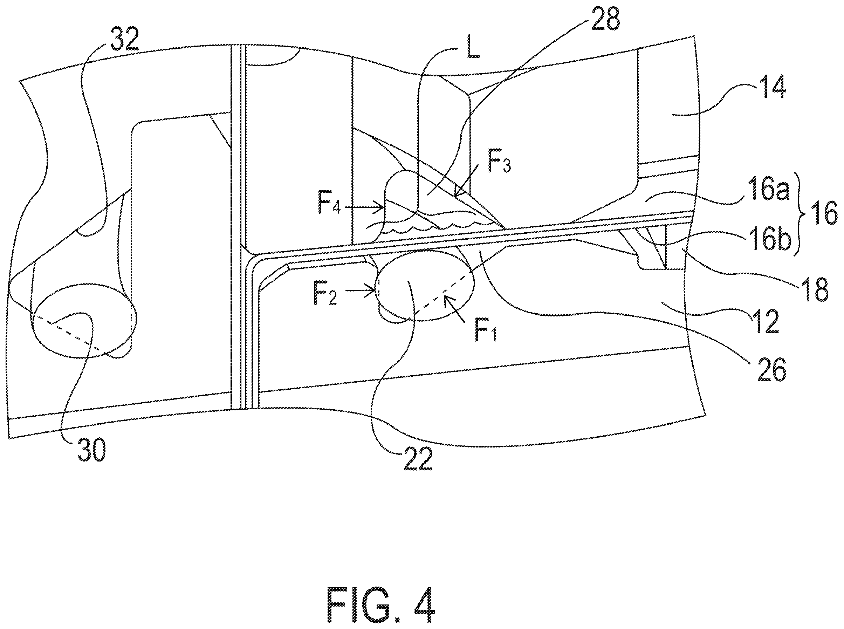

A second portion of the sensor probe 16 is configured to be electrically connected to the one or more electronic circuits within the housing formed by the base 12 and the cover 14, as shown in FIG. 2. The second portion of the sensor probe 16 is shown as extending in a generally horizontal direction in FIG. 2. A length section (L in FIG. 4) of the second portion of the sensor probe 16 extends through the seal barrier in the housing formed by the base 12 and the cover 14, to enable the second portion of the sensor probe 16 to be electrically coupled to the one or more electronic circuits 18, while maintaining a sealed environment for the one or more electronic circuits 18 and other components within the housing.

In particular examples described below, the one or more substrates of the sensor probe 16 are configured to provide an enhanced seal through the seal barrier. The enhanced seal inhibits passage of moisture or other contaminants from the environment external to the housing formed by the base 12 and the cover 14, to the environment within the housing in which the one or more electronic circuits 18 and one or more batteries 20 or other power sources are contained.

The base 12 and the cover 14 may be made of any suitable material, such as, but not limited to plastic, metal, ceramic, composite material, or the like. In particular examples, the base 12 and the cover are generally rigid structures. In other examples, the base 12 may have sufficient rigidity to hold its shape, but may be made of material having some flexibility or malleability, such as but not limited to a soft plastic, a rubber or rubber-like material, or the like, for patient comfort. In particular examples, the base 12 and the cover 14 have mating configurations that enable the base 12 and the cover 14 to be selectively coupled together to form a housing, and to be selectively de-coupled from the coupled state, by applying pressure manually or with a tool. Such configurations may include, but are not limited to friction-fitting configurations, snap-fitting configurations, threaded configurations or the like. In other examples, the base 12 and the cover 14 are configured to be coupled and/or decoupled, by other suitable coupling mechanisms including, but not limited to threaded fasteners, clamps, adhesive material, or combinations thereof.

The skin-facing surface 12a of the base 12 is configured to secure to the skin of a patient as shown in FIG. 1. One or more mechanisms may be provided for securing the surface 12a of the base 12 to the patient such as, but not limited to adhesive on the surface 12a, suture tabs on the base 12 for suturing the base to the skin S, straps extending from the base for wrapping around an arm, leg, torso or other portion of the patient, or the like. In the example in FIG. 1, an adhesive material 22 is provided on the surface 12a of the base 12, to adhere the base 12 to the patient's skin.

The one or more electronic circuits 18 may include processing electronics configured to process or analyze information provided by the sensor probe 16 and to provide sensor data signals associates with the information, operational memory associated with the processing electronics, wireless transmission electronics configured to transmit (wirelessly) data signals associated with information provided by the sensor probe, other suitable electronics or combinations thereof. In certain examples, the device 10 may also include one or more infusion tubes (not shown) connected to an infusion media supply port or reservoir (not shown) within the housing, to deliver infusion media to the patient.

The sensor probe 16 may include one, two or more substrates, each formed in a thin film or a relatively thin, strip-like shape that is flexible along its longitudinal dimension. In other examples, the one or more substrates may have other suitable shapes including, but not limited to cylindrical, or semi-cylindrical shapes. In the example in Figs. 1-3, the sensor probe 16 has two substrates 16a and 16b (best shown in FIG. 3). At least one of the substrates includes an electrically non-conductive support material on which one or more electrically conductive members or components are formed or supported. The support material may be any suitable electronic substrate material that is compatible with a subcutaneous environment such as, but not limited to a thin film polyimide, polyethylene or other suitable material.

The conductive members or components on at least one of the substrates electrically couple to the one or more electronic circuits 18 in the housing, to form an analyte sensor for sensing one or more defined analytes as described herein. In certain examples, the analyte includes blood glucose concentration, and the first portion of at least one of the substrates holds a glucose oxidase enzyme in relation to one or more electrical contacts, to monitor blood glucose levels in the patient by effecting a reaction of glucose and oxygen, when the sensor device is in the operational state shown in FIG. 1. Examples of blood glucose sensors and substrates of such sensors are described above. However, in other examples, the substrate sealing concepts and technology described herein may be adapted for use with any one of a wide variety of other sensors or other types of devices having one or more substrates that pass through a seal barrier.

Once the sensor device 10 is secured to the patient in the operational state as shown in FIG. 1, the sensor probe 16 and the electronics 18 may sense or monitor one or more defined analytes (such as, but not limited to blood glucose concentration) of the patient and produce one or more sensor data signals associated with the one or more analytes. In certain examples, the electronics 18 of the sensor device may be configured to transmit the one or more sensor data signals to an external processing device such as, but limited to a computer, mobile processor, or other processing device (not shown). In some examples, the sensor device 10 may be configured for continuous monitoring of the analyte over a period of time. In other examples, the sensor device 10 may be configured to provide one or more intermittent or separate sensing operations in sequence such as, but not limited to a periodic sequence.

The electronics 18 may use wireless data communication techniques to transmit data indicative of sensed blood glucose levels or another analyte to a receiving device such as a portable infusion pump, a glucose monitor device, and/or the like. For example, the transmitted sensor signal may be used to generate a controller input for a controller to generate commands that affect the operation of a delivery system to infuse a medium such as, but not limited to insulin, into the patient.

In the operational state shown in FIG. 1, the sensor probe 16 extends through an opening 12b (shown in FIG. 3) in the base 12, such that a first portion of the sensor probe 16 extends from a skin-facing surface 12a of the base 12, and a second portion of the sensor probe 16 is located within the housing defined by the base 12 and the cover 14. As shown in FIG. 1, the first portion of the sensor probe 16 may extend from a skin-facing surface 12a of the base 12, through the patient's skin S, to a transcutaneous depth D within the patient. The transcutaneous depth D of the sensor probe 16 is defined by the length of the first portion of the sensor probe 16 from the skin-facing surface 12a of the base 12, and the angle of insertion of the sensor probe 16. In the example of FIGS. 1-3, the first portion of the sensor probe 16 extends generally perpendicular to the skin-facing surface 12a of the base 12 (and to the external surface of the skin). In other examples, the first portion of the sensor probe 16 may extend at any suitable oblique angle relative to the skin-facing surface 12a of the base 12 (and to the external surface of the skin).

As shown in FIGS. 2 and 3, the second portion of the sensor probe 16 includes a length section that extends (in its longitudinal dimension) at an angle, transverse to the longitudinal dimension of the first portion of the sensor probe. In the example in FIGS. 1-3, that angle is substantially perpendicular. In other examples, the angle formed between the first portion and the transverse length section of the second portions of the sensor probe is oblique. The angle enables the housing of the base 12 and the cover 14 to be formed with a relatively thin dimension.

To avoid leakage of fluids through the opening 12b in the base and into one or more volumes of the housing that contain the electronics 18 and the power source 20, the sensor device 10 includes a seal configuration that provides a seal barrier between the external environment and the one or more volumes of the housing. In the example in FIGS. 1-3, the seal configuration includes a first O-ring 22 and a second O-ring 24. Each O-ring 22 and 24 may have a generally toroidal shape and may be made of an elastic material such as, but not limited to nitrile, neoprene, silicone, polytetrafluoroethylene (PTFE), ethylene propylene or other suitable rubber or polymer material. In other examples, other suitable seal members or material may be employed as an alternative to (or in addition to) the O-rings 22 and 24 including, but not limited to, other annular-shaped or non-annular seal members, flowable sealing materials, combinations thereof, or the like.

The first O-ring 22 resides in a first annular cavity 26 located in the base 12. The second O-ring 24 resides in a second annular cavity 28 located in the cover 24. The size and shape of the cavities 26 and 28 relative to the size of the O-rings 22 and 24 are configured to enable a portion of each O-ring to protrude out (at least slightly) from the open side of its associated cavity. However, the open sides of the annular cavities 26 and 28 are arranged to align with each other when the base 12 and the cover 14 are coupled to each other. In that manner, the protruding portions of the first and second O-rings 22 and 24 are arranged to engage and compress against each other to form a sealed barrier between the base 12 and the cover 14, when the base 12 and the cover 14 are coupled to each other.

In particular examples, each of the first and second annular cavities 26 and 28 is configured to apply one or more forces on the associated O-rings 22 and 24 when the base 12 and the cover 14 are coupled to each other, to force the O-rings 22 and 24 in a direction toward each other and enhance compression of the O-rings against each other. An example of cavity configurations for the first and second annular cavities 26 and 28 is shown in FIG. 2 and in an enlarged partial view in FIG. 4 (where FIG. 4 shows an enlarged view of the portion of FIG. 2 labeled 4). However, the upper O-ring 24 is omitted from the cavity 28 in FIG.4, to enable viewing of the lower O-ring 22 and the length section of the sensor probe 16 that extends between the O-rings 22 and 24 and through the seal barrier provided by the O-rings. Also, the uncompressed shape of the O-rings 22 and 24 is shown in the drawings. However, when the base 12 and the cover 14 are coupled together, the surfaces of the cavities 26 and 28 press against the O-rings to compress the O-rings together and against the sensor probe 16 by compression forces described below.

In the example in FIGS. 2 and 4, each of the first and second annular cavities 26 and 28 has an angled wall and defines a cross-section shape that tapers from a narrower dimension in the depth of the cavity, to a wider dimension toward the open side of the cavity. With reference to FIG. 4, the first annular cavity 26 has an angled wall 30, and the second annular cavity 28 has an angled wall 32. Each angled wall 30 and 32 defines an oblique angle relative to the second portion of the sensor probe 16. Each O-ring 22 and 24 may also abut at least one further walls in each cavity, which may or may not be at an oblique angle relative to the sensor probe 16.

When the base 12 and the cover 14 are coupled to each other, the angled or tapered walls 30 and 32 of the cavities 26 and 28 abut the O-rings 22 and 24 and force the O-rings toward and against each other, and against the walls of the cavities 26 and 28, to form a seal barrier for a fluid seal between the two O-rings 22 and 24, and between each O-ring and the walls of its associated cavity 26 and 28, as shown in FIGS. 2 and 4. For example, when the base 12 and the cover 14 are coupled to each other compression forces F₁ and F₂ are imparted on the O-ring 22 (and Forces F₃ and F₄ on the O-ring 24, if included in the annular cavity 28). The combination of the forces F₁ and F₂ (and of the forces F₃ and F₄) press the O-ring's 22 and 24 toward each other, and compress the O-rings against each other and against the length section L of the sensor probe 16 that passes between the O-rings, to provide a seal as described herein.

Thus, in examples described herein, a length section L of the one or more substrates of the sensor probe 16 extends through the seal barrier, by extending across and between the two O-rings 22 and 24, as shown in FIGS. 2 and 4. In that arrangement, the O-rings 22 and 24 may be pressed toward each other with sufficient force to form a fluid seal against and around the section of the length section L of the sensor probe 16 that extends between the two O-rings 22 and 24. However, according to certain examples described herein, the sealing effect of the O-rings 22 and 24 is enhanced by providing an tortuous edge pattern (such as, but not limited to an undulating edge wave pattern) on one or each substrate of the sensor probe 16, at least along the length section L of the substrate that passes between the two O-rings 22 and 24. In other examples, other suitable edge shape patterns (differing from a straight edge) may be employed to enhance the sealing effect of the O-rings 22 and 24.

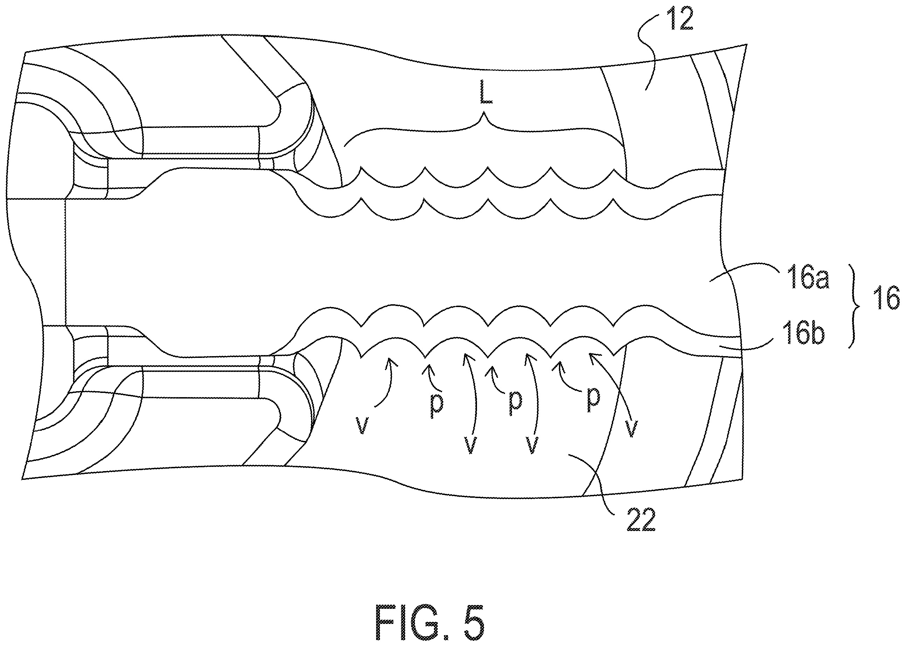

An example of an edge wave pattern is shown on the further side edge (in the perspective view) of the length section L of the sensor probe 16 FIG. 4. Another view of the example edge wave pattern on the sensor probe 16 is shown in FIG. 5 which, relative to FIG. 4, is a downward-directed view of the length section L of the sensor probe 16 and a portion of the lower O-ring 22 (with the upper O-ring 24 omitted). In the example in FIG. 5, both side edges of each of the substrates 16a and 16b of the sensor probe 16 has an edge wave pattern along the length portion L. In other examples, only one of the substrates 16a and 16b has the edge wave pattern along the length section L. In those or other examples, only one of the two edges of one or both substrates 16a and 16b has the edge wave pattern along the length L.

In particular examples, the edge wave pattern (or other tortuous edge pattern) defines one or more (or a plurality of) peaks P and one or more (or a plurality of) valleys V. A peak P on an edge may be located between two valleys V on that edge. A valley V on an edge may be located between two peaks P on that edge. In the example in FIG. 5, both side edges of the sensor probe 16 have an edge wave pattern that has multiple peaks P and multiple valleys V. In the example in FIG. 5, each valley V has a relatively smooth curve shape and each peak P has a relatively sharp or pointed shape, which can enhance sealing effects along the edge as described herein. In other examples, the valleys V and the peaks P may have other suitable shapes. For example, one or more (or each) of the peaks P may have a more rounded tip than represented in FIG. 5.

In other examples, the sensor probe 16 may have an edge wave pattern on one, but not both side edges, for enhanced sealing on one of the side edges. In other examples, the edge wave pattern may have a single peak P or a single valley V, or may have any other number of peaks P and valleys V. In particular examples, the valleys in the edge wave pattern have a smooth curvature extending to at least one peak P. In other examples, the peaks and valleys may be formed with straight lines, in a flat-tooth pattern or in a saw-tooth wave pattern. Other examples may include other undulating edge patterns that include one or more peaks or one or more valleys (or both).

In the example shown in FIG. 5, the substrates 16a and 16b have the same edge wave pattern on both edges. However, the substrate 16b is wider (from side edge to side edge) than the substrate 16a. Accordingly, when the substrates 16a and 16b are arranged together as shown in Figs. 4 and 5, each outer edge of the substrate 16b extends beyond the corresponding outer edge of the substrate 16a. In further examples, the substrate 16a may be wider than the substrate 16b, or they may have substantially equal widths. In further examples, the pattern or pitch of the wave can vary along one or both substrates.

However, in FIG. 5, the edge wave patterns of the substrates 16a and 16b are aligned such that the peaks P of the edge wave pattern of substrate 16a are aligned with and overlap the peaks P of the edge wave pattern of substrate 16b. Similarly, the valleys V the edge wave pattern of substrate 16a are aligned with and overlap the valleys V of the edge wave pattern of substrate 16b. In other examples, the peaks P of the substrate 16a may be arranged to align with and overlap the valleys V of the substrate 16b, and vice versa. In further examples, the edge-to-edge width of the substrates 16a and 16b may be substantially the same.

As compared to a straight cut edge, the edge wave pattern enhances the fluid seal between the O-rings 22 and 24 and the edges of the substrate. The peaks P of the waves create higher pressure points on the opposing O-ring seals (relative to a straight cut edges), enabling the O-rings to better close a potential gap between the seals at the side edges of the substrates. The wave shape also allows the O-ring seals to compress and wrap around the tip of the wave, sealing in multiple directions. Also, due to the wave shape, a potential leak path along the length dimension of the wave-cut edge is non-linear, helping to inhibit forward progression of a leak. Also, the wave shape (or other shape defining a tortuous path along the side edges of the substrates) can create interruptions along paths to reduce pressure and inhibit forward propagation of an edge leak. An edge wave pattern having multiple wave tips creates redundant enhanced seal points in the sensor device 10.

The edge cut pattern on the edge or edges of the substrates 16a and 16b may be formed by any suitable manufacturing process. In typical processes of cutting substrates from a sheet of substrate material, such as by laser cutting or other suitable cutting techniques, the manufacturing systems may be configured to form a wave pattern (or other pattern) when cutting or otherwise forming side edges of the substrate (edges that are configured to cross a seal barrier in a device). In other examples, the edge cut pattern can be formed by other suitable manufacturing techniques including, but not limited to machining, molding, mechanical cutting, slitting, application of heat, or the like.

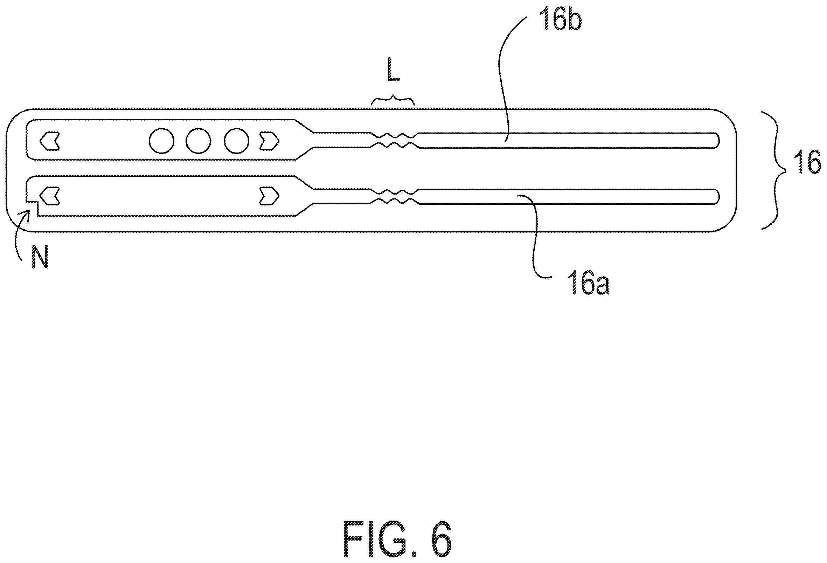

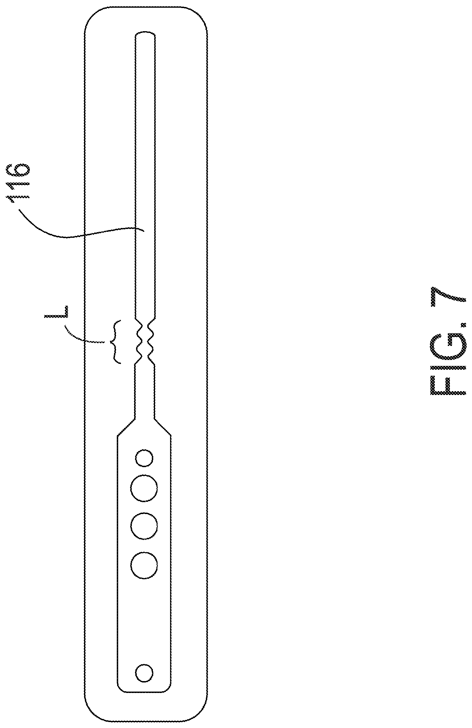

An example of substrates 16a and 16b of the sensor probe 16 is shown in FIG. 6, after the substrates have been cut or otherwise formed from a substrate material, and before they are bent to the angled shape shown in FIGS. 2-4. As shown in FIG. 6, the side edges of the length section L of each of the substrates 16a and 16b has an edge wave pattern. Another example of a sensor probe 116 is shown in FIG. 7, where the sensor probe 116 has a single substrate. The substrate of the sensor probe 116 has an edge wave pattern on each of the side edges of the length section L, similar to the substrates in FIG. 6.

The substrate 16b of the sensor probe 16 and the substrate of the sensor probe 116 contain or support one or more electrically conductive members or components that form part of the electronics of a sensor. The one or more electrically conductive members or components may include, but are not limited to electrical leads, electrical traces, electrical contacts, electronic devices or combinations thereof. In certain examples, the substrates may include a coating of a drug or other treatment substance that treats the patient when the sensor probe 16 or 116 is in the operational state (as shown in FIG. 1).

In the example in FIG. 6, the substrate 16a may include a coating of a drug or other treatment substance. In particular examples, the substrate 16a does not include electrically conductive members or components of the sensor. In certain examples, the substrate 16a includes the coating of a drug or other treatment substance on one side of the substrate (for example, the side facing outward of the page in FIG. 6), but not on the other side (for example, the side out of view and facing inward of the page in FIG. 6). Accordingly, the amount of drug or other treatment substance may be reduced (and the cost and manufacturing steps may be reduced), relative to coating two sides of the substrate. In those examples, a notch N or other marking or visible shape may be formed into one corner of the substrate 16b, to identify the coated side of the substrate. When assembled in the sensor device 10, the substrates 16a and 16b are arranged adjacent and facing each other, with the uncoated side of the substrate 16b facing a side of the substrate 16a and with the coated side of the substrate 16b facing away from the substrate 16a.

The sensor device 10 may be assembled in any suitable manner. In one example, with reference to FIG. 3, a manufacturing method involves providing the base 12 and the cover 14 by molding or other suitable manufacturing process. Then, components may be arranged on the base 12, in a relatively simple stacking assembly. For example, one or more electronic circuits 18 may be arranged on the base 12. In addition, the first O-ring 22 may be arranged in the annular recess 26 of the base 12, and placed over and surrounding the opening 12b in the base 12.

Then the sensor probe 16 may be arranged with the first portion of the sensor probe 16 extending through the opening in the first O-ring 22 and the opening 12b in the base, and with the second portion of the sensor probe extending transverse to (at a perpendicular or oblique angle relative to) the first portion of the sensor probe 16. The second portion of the sensor probe 16 may be arranged to at least partially overlap and lay on top of the one or more electronic circuits 18 and has one or more electrical conductors or components that are electrically connected to the one or more electronic circuits 18. In other examples, the second portion of the sensor probe 16 may be arranged adjacent the one or more electronic circuits and be electrically connected to the one or more electronic circuits 18 without overlapping or laying on top of the electronic circuits. In examples in which the sensor probe 16 has two (or more) substrates 16a and 16b, the two (or more) substrates may be arranged separately and individually on the base 12, as described above. In other examples, the two (or more) substrates 16a and 16b may be placed together and then arranged (together) on the base 12, as described above.

Once the first O-ring and the sensor probe 16 are arranged on the base 12 as described above, the second O-ring 24 is placed on and in alignment with the first O-ring 22, with the length section L of the second portion of the sensor probe 16 sandwiched between the first O-ring 22 and the second O-ring 24. One or more other components may be placed or stacked on the above-noted components, including but not limited to batteries 20 or other power sources, additional electronic circuits or components 26, or the like.

Once other components are arranged as described above, the cover 14 may be placed over the base 12 and coupled to the base 12, to encase the other components within the housing formed by the base 12 and the cover 14. In other examples, the second O-ring 24 may be placed in the annular recess 28 in the cover 14, and then stacked onto the first O-ring 22 by coupling the cover 14 to the base 12.

Once the sensor device 10 is assembled, the sensor device may be packaged or stored for future use, or may be inserted on a patient for operation. The assembled sensor device 10 may be inserted and secured to a patient manually or with an insertion tool as described above. In other examples, the insertion tool may complete part of the assembly by, for example moving the sensor probe 16 through the first O-ring 22 and the opening 12b in the base and moving the cover 14 onto the base 12, as the sensor probe 16 is moved toward an operational state, inserted in the patient.

The assembled sensor device 10, having one or more sensor substrates extending through the seal barrier formed by the O-rings 22 and 24 has an edge cut pattern as described herein, to enhance the sealing effect of the seal barrier. The edge cut pattern can enable the use of standard O-ring components, while also achieving a high process output, with high product manufacturing yields. Accordingly, the edge cut pattern on the one or more substrates can provide significant improvements in manufacturing and operation of the sensor device 10.

In other examples, other seal members or materials may be employed as an alternative to or in addition to the O-rings 22 and 23. Also, while the above examples employ sensor substrates, other examples may include other sensor devices or other devices that employ one or more substrate that passes through the wall of an enclosure, at the location of the seal. In those other examples, the section of the substrate with an edge cut pattern is sandwiched between two O-rings (or two other seal members or materials) placed in axial compression, to provide an enhanced seal barrier as described herein.

While various exemplary embodiments have been presented in the foregoing detailed description, it should be appreciated that a vast number of variations exist. It should also be appreciated that the exemplary embodiment or embodiments described herein are not intended to limit the scope, applicability, or configuration of the claimed subject matter in any way. Rather, the foregoing detailed description will provide those skilled in the art with a convenient road map for implementing the described embodiment or embodiments. It should be understood that various changes can be made in the function and arrangement of elements without departing from the scope defined by the claims .

## Claims

1. A sensor device (10) comprising:
a housing configured to be secured to a patient;
a seal structure located within the housing; and
a sensor probe (16) having at least one substrate (16a) supported by the housing, the substrate having a first length portion and a second length portion, the first length portion being external to the housing and configured to be inserted into the patient when the housing is secured to the patient, the second length portion being in or on the housing and having a section that extends through the seal structure;
wherein the section of the second length portion of the at least one substrate that extends through the seal structure has at least one side edge having a tortuous edge pattern that enhances a seal effect of the seal structure at the section of the second length portion of the at least one substrate.

2. The sensor device of claim 1, wherein the section of the second length portion of the sensor substrate that extends through the seal structure has two side edges, each having a tortuous edge pattern.

3. The sensor device of claim 1 or 2, wherein the tortuous edge pattern comprises a wave pattern having a plurality of peaks (P) or a plurality of valleys (V) on each of two side edges,
and/or
wherein the tortuous edge pattern on at least one side edge comprises a wave pattern having a plurality of peaks and a plurality of valleys.

4. The sensor device of any of the claims 1 to 3, wherein the seal structure comprises first and second O-rings (22, 24), and the section of the second length portion of the sensor substrate extends between the first and the second O-rings.

5. The sensor device of claim 4, wherein the housing comprises a base (12) and a cover (14), and wherein the sensor device comprises a stacked arrangement including the first O-ring arranged on the base, the section of the second length portion of the sensor substrate arranged on the first O-ring, the second O-ring arranged on the section of the second length portion of the sensor substrate, and the cover arranged on the second O-ring.

6. The sensor device of claim 4 or 5, wherein the housing includes a base (12) and a cover (14), wherein the base has a first annular cavity (26) in which the first O-ring resides, and the cover has a second annular cavity (28) in which the second O-ring resides.

7. The sensor device of claim 6, wherein at least one of the first and second annular cavities has a cross-section shape that tapers from a narrower dimension toward a depth of the cavity, to a wider dimension toward an open side of the cavity to enhance compression of the O-ring residing therein, when the base and the cover are coupled together
and/or
wherein each of the first and second annular cavities has a cross-section shape that tapers from a narrower dimension toward a depth of the cavity, to a wider dimension toward an open side of the cavity to enhance compression of the O-rings, when the base and the cover are coupled together.

8. The sensor device of any of the claims 1 to 7, wherein the at least one substrate of the sensor probe comprises a first substrate having one or more electrical conductors or electrical components.

9. The sensor device of claim 8, wherein the at least one substrate of the sensor probe comprises a second substrate having one side provided with a coating of a drug or treatment substance, the one side of the second substrate facing away from the first substrate of the sensor probe.

10. The sensor device of claim 9, wherein the second substrate has a mark or other indicia that identifies the one side provided with the coating of the drug or treatment substance,
and/or
wherein the second substrate has a second side that faces the first substrate of the sensor probe, the second side of the second substrate being uncoated.

11. A sensor probe (16) configured for the use in the sensor device (10) according to any of the claims 1 to 10, wherein the sensor probe (16) has at least one substrate (16a) supported by the housing, the substrate having the first length portion and the second length portion, the first length portion being external to the housing and configured to be inserted into the patient when the housing is secured to the patient, the second length portion being in or on the housing and having a section that extends through the seal structure, wherein the section of the second length portion of the at least one substrate that extends through the seal structure has at least one side edge having a tortuous edge pattern that enhances a seal effect of the seal structure at the section of the second length portion of the at least one substrate.

12. The sensor probe of claim 11, wherein the tortuous edge pattern on at least one side edge comprises a wave pattern having a plurality of peaks or a plurality of valleys.

13. A method of making a sensor device (10), the method comprising:
providing a housing configured to be secured to a patient;
arranging a seal structure within the housing; and
supporting a sensor probe (16) having at least one substrate (16a) by the housing, the substrate having a first length portion and a second length portion, the first length portion being external to the housing and configured to be inserted into the patient when the housing is secured to the patient, the second length portion being in or on the housing and having a section that extends through the seal structure;
wherein the section of the second length portion of the at least one substrate that extends through the seal structure has at least one side edge having an undulating edge pattern that enhances a seal effect of the seal structure at the section of the second length portion of the at least one substrate, particularly
wherein the undulating edge pattern on the at least one side edge comprises a wave pattern having a plurality of peaks or a plurality of valleys.

14. The method of claim 13, wherein arranging a seal structure within the housing comprises:
arranging a first O-ring (22) on a base (12) of the housing, the first O-ring having a central opening;
arranging a portion of the at least one substrate to extend through the opening in the first O-ring; and
arranging the second O-ring (24) on the first O-ring, with a central opening of the second O-ring in alignment with the central opening first O-ring; and
extending a section of a second portion of the at least one substrate between the first O-ring and the second O-ring.

15. The method of claim 13 or 14, wherein:
the housing includes a base (12) and a cover (14) configured to couple together;
arranging a first O-ring (22) comprises locating the first O-ring in a first annular cavity (26) in the base;
arranging the second O-ring (24) comprises locating the second O-ring in a second annular cavity (28) in the cover;
at least one of the first and second annular cavities has a cross-section shape that tapers from a narrower dimension toward a depth of the cavity, to a wider dimension toward an open side of the cavity to enhance compression of the O-ring residing therein, when the base and the cover are coupled together.

## Patentansprüche

1. Sensorvorrichtung (10), umfassend:
ein Gehäuse, das konfiguriert ist, um an einem Patienten befestigt zu werden;
eine Dichtungsstruktur, die sich innerhalb des Gehäuses befindet; und
eine Sensorsonde (16), die mindestens ein Substrat (16a) aufweist, das durch das Gehäuse getragen wird, wobei das Substrat einen ersten Längenabschnitt und einen zweiten Längenabschnitt aufweist, wobei sich der erste Längenabschnitt außerhalb des Gehäuses befindet und konfiguriert ist, um in den Patienten eingeführt zu werden, wenn das Gehäuse an dem Patienten befestigt ist, sich der zweite Längenabschnitt in oder auf dem Gehäuse befindet und einen Bereich aufweist, der sich durch die Dichtungsstruktur hindurch erstreckt;
wobei der Bereich des zweiten Längenabschnitts des mindestens einen Substrats, der sich durch die Dichtungsstruktur hindurch erstreckt, mindestens eine Seitenkante aufweist, die ein gewundenes Kantenmuster aufweist, das eine Dichtungswirkung der Dichtungsstruktur an dem Bereich des zweiten Längenabschnitts des mindestens einen Substrats verstärkt.

2. Sensorvorrichtung nach Anspruch 1, wobei der Bereich des zweiten Längenabschnitts des Sensorsubstrats, der sich durch die Dichtungsstruktur hindurch erstreckt, zwei Seitenkanten aufweist, die jeweils ein gewundenes Kantenmuster aufweisen.

3. Sensorvorrichtung nach Anspruch 1 oder 2, wobei das gewundene Kantenmuster ein Wellenmuster umfasst, das eine Vielzahl von Spitzen (P) oder eine Vielzahl von Tälern (V) auf jeder der zwei Seitenkanten aufweist,
und/oder
wobei das gewundene Kantenmuster auf mindestens einer Seitenkante ein Wellenmuster umfasst, das eine Vielzahl von Spitzen und eine Vielzahl von Tälern aufweist.

4. Sensorvorrichtung nach einem der Ansprüche 1 bis 3, wobei die Dichtungsstruktur einen ersten und einen zweiten O-Ring (22, 24) umfasst und sich der Bereich des zweiten Längenabschnitts des Sensorsubstrats zwischen dem ersten und dem zweiten O-Ring erstreckt.

5. Sensorvorrichtung nach Anspruch 4, wobei das Gehäuse eine Basis (12) und eine Abdeckung (14) umfasst und wobei die Sensorvorrichtung eine gestapelte Anordnung umfasst, die den ersten O-Ring, der auf der Basis angeordnet ist, den Bereich des zweiten Längenabschnitts des Sensorsubstrats, der auf dem ersten O-Ring angeordnet ist, den zweiten O-Ring, der auf dem Bereich des zweiten Längenabschnitts des Sensorsubstrats angeordnet ist, und die Abdeckung einschließt, die auf dem zweiten O-Ring angeordnet ist.

6. Sensorvorrichtung nach Anspruch 4 oder 5, wobei das Gehäuse eine Basis (12) und eine Abdeckung (14) einschließt, wobei die Basis einen ersten ringförmigen Hohlraum (26) aufweist, in dem sich der erste O-Ring befindet, und die Abdeckung einen zweiten ringförmigen Hohlraum (28) aufweist, in dem sich der zweite O-Ring befindet.

7. Sensorvorrichtung nach Anspruch 6, wobei mindestens einer des ersten und des zweiten ringförmigen Hohlraums eine Querschnittsform aufweist, die sich von einer schmaleren Abmessung zu einer Tiefe des Hohlraums hin zu einer breiteren Abmessung zu einer offenen Seite des Hohlraums hin verjüngt, um eine Kompression des darin befindlichen O-Rings zu verstärken, wenn die Basis und die Abdeckung miteinander gekoppelt sind
und/oder
wobei jeder des ersten und des zweiten ringförmigen Hohlraums eine Querschnittsform aufweist, die sich von einer schmaleren Abmessung zu einer Tiefe des Hohlraums hin zu einer breiteren Abmessung zu einer offenen Seite des Hohlraums hin verjüngt, um die Kompression der O-Ringe zu verstärken, wenn die Basis und die Abdeckung miteinander gekoppelt sind.

8. Sensorvorrichtung nach einem der Ansprüche 1 bis 7, wobei das mindestens eine Substrat der Sensorsonde ein erstes Substrat umfasst, das einen oder mehrere elektrische Leiter oder eine oder mehrere elektrische Komponenten aufweist.

9. Sensorvorrichtung nach Anspruch 8, wobei das mindestens eine Substrat der Sensorsonde ein zweites Substrat umfasst, das eine Seite aufweist, die mit einer Beschichtung eines Arzneimittels oder einer Behandlungssubstanz versehen ist, wobei die eine Seite des zweiten Substrats von dem ersten Substrat der Sensorsonde abgewandt ist.

10. Sensorvorrichtung nach Anspruch 9, wobei das zweite Substrat eine Markierung oder ein anderes Kennzeichen aufweist, das die eine Seite identifiziert, die mit der Beschichtung des Arzneimittels oder der Behandlungssubstanz versehen ist,
und/oder
wobei das zweite Substrat eine zweite Seite aufweist, die dem ersten Substrat der Sensorsonde zugewandt ist, wobei die zweite Seite des zweiten Substrats unbeschichtet ist.

11. Sensorsonde (16), die für die Verwendung in der Sensorvorrichtung (10) nach einem der Ansprüche 1 bis 10 konfiguriert ist, wobei die Sensorsonde (16) mindestens ein Substrat (16a) aufweist, das durch das Gehäuse getragen wird, wobei das Substrat den ersten Längenabschnitt und den zweiten Längenabschnitt aufweist, wobei sich der erste Längenabschnitt außerhalb des Gehäuses befindet und konfiguriert ist, um in den Patienten eingeführt zu werden, wenn das Gehäuse an dem Patienten befestigt ist, sich der zweite Längenabschnitt in oder auf dem Gehäuse befindet und einen Bereich aufweist, der sich durch die Dichtungsstruktur hindurch erstreckt, wobei der Bereich des zweiten Längenabschnitts des mindestens einen Substrats, der sich durch die Dichtungsstruktur hindurch erstreckt, mindestens eine Seitenkante aufweist, die ein gewundenes Kantenmuster aufweist, das eine Dichtungswirkung der Dichtungsstruktur an dem Bereich des zweiten Längenabschnitts des mindestens einen Substrats verstärkt.

12. Sensorsonde nach Anspruch 11, wobei das gewundene Kantenmuster auf mindestens einer Seitenkante ein Wellenmuster umfasst, das eine Vielzahl von Spitzen oder eine Vielzahl von Tälern aufweist.

13. Verfahren zum Herstellen einer Sensorvorrichtung (10), das Verfahren umfassend:
Bereitstellen eines Gehäuses, das konfiguriert ist, um an einem Patienten befestigt zu werden;
Anordnen einer Dichtungsstruktur innerhalb des Gehäuses; und
Tragen einer Sensorsonde (16), die mindestens ein Substrat (16a) aufweist, durch das Gehäuse, wobei das Substrat einen ersten Längenabschnitt und einen zweiten Längenabschnitt aufweist, wobei sich der erste Längenabschnitt außerhalb des Gehäuses befindet und konfiguriert ist, um in den Patienten eingeführt zu werden, wenn das Gehäuse an dem Patienten befestigt ist, sich der zweite Längenabschnitt in oder auf dem Gehäuse befindet und einen Bereich aufweist, der sich durch die Dichtungsstruktur hindurch erstreckt;
wobei der Bereich des zweiten Längenabschnitts des mindestens einen Substrats, der sich durch die Dichtungsstruktur hindurch erstreckt, mindestens eine Seitenkante aufweist, die ein wellenförmiges Kantenmuster aufweist, das eine Dichtungswirkung der Dichtungsstruktur an dem Bereich des zweiten Längenabschnitts des mindestens einen Substrats verstärkt, insbesondere
wobei das wellenförmige Kantenmuster auf der mindestens einen Seitenkante ein Wellenmuster umfasst, das eine Vielzahl von Spitzen oder eine Vielzahl von Tälern aufweist.

14. Verfahren nach Anspruch 13, wobei das Anordnen einer Dichtungsstruktur innerhalb des Gehäuses umfasst:
Anordnen eines ersten O-Rings (22) auf einer Basis (12) des Gehäuses, wobei der erste O-Ring eine zentrale Öffnung aufweist;
Anordnen eines Abschnitts des mindestens einen Substrats, um sich durch die Öffnung in dem ersten O-Ring hindurch zu erstrecken; und
Anordnen des zweiten O-Rings (24) auf dem ersten O-Ring, wobei eine zentrale Öffnung des zweiten O-Rings mit der zentralen Öffnung des ersten O-Rings ausgerichtet ist; und
Erstrecken eines Bereichs eines zweiten Abschnitts des mindestens einen Substrats zwischen dem ersten O-Ring und dem zweiten O-Ring.

15. Verfahren nach Anspruch 13 oder 14, wobei:
das Gehäuse eine Basis (12) und eine Abdeckung (14) einschließt, die konfiguriert sind, um miteinander zu koppeln;
das Anordnen eines ersten O-Rings (22) ein Platzieren des ersten O-Rings in einem ersten ringförmigen Hohlraum (26) in der Basis umfasst;
das Anordnen des zweiten O-Rings (24) das Platzieren des zweiten O-Rings in einem zweiten ringförmigen Hohlraum (28) in der Abdeckung umfasst;
mindestens einer des ersten und des zweiten ringförmigen Hohlraums eine Querschnittsform aufweist, die sich von einer schmaleren Abmessung zu einer Tiefe des Hohlraums hin zu einer breiteren Abmessung zu einer offenen Seite des Hohlraums hin verjüngt, um die Kompression des darin befindlichen O-Rings zu verstärken, wenn die Basis und die Abdeckung miteinander gekoppelt sind.

## Revendications

1. Dispositif capteur (10) comprenant :
un boîtier conçu pour être fixé à un patient ;
une structure d'étanchéité située à l'intérieur du boîtier ; et
une sonde de capteur (16) ayant au moins un substrat (16a) supporté par le boîtier, le substrat ayant une première partie de longueur et une seconde partie de longueur, la première partie de longueur étant externe au boîtier et conçue pour être insérée dans le patient lorsque le boîtier est fixé au patient, la seconde partie de longueur étant dans ou sur le boîtier et ayant une section qui s'étend à travers la structure d'étanchéité ;
dans lequel la section de la seconde partie de longueur de l'au moins un substrat qui s'étend à travers la structure d'étanchéité a au moins un bord latéral ayant un motif de bord tortueux qui renforce un effet d'étanchéité de la structure d'étanchéité au niveau de la section de la seconde partie de longueur de l'au moins un substrat.

2. Dispositif capteur selon la revendication 1, dans lequel la section de la seconde partie de longueur du substrat de capteur qui s'étend à travers la structure d'étanchéité a deux bords latéraux, chacun ayant un motif de bord tortueux.

3. Dispositif capteur selon la revendication 1 ou 2, dans lequel le motif de bord tortueux comprend un motif de vague ayant une pluralité de crêtes (P) ou une pluralité de vallées (V) sur chacun des deux bords latéraux,
et/ou
dans lequel le motif de bord tortueux sur au moins un bord latéral comprend un motif de vague ayant une pluralité de crêtes et une pluralité de vallées.

4. Dispositif capteur selon l'une quelconque des revendications 1 à 3, dans lequel la structure d'étanchéité comprend un premier et un second joint torique (22, 24), et la section de la seconde partie de longueur du substrat de capteur s'étend entre le premier et le second joint torique.

5. Dispositif capteur selon la revendication 4, dans lequel le boîtier comprend une base (12) et un couvercle (14), et dans lequel le dispositif capteur comprend un agencement empilé comportant le premier joint torique agencé sur la base, la section de la seconde partie de longueur du substrat de capteur agencée sur le premier joint torique, le second joint torique agencé sur la section de la seconde partie de longueur du substrat de capteur, et le couvercle agencé sur le second joint torique.

6. Dispositif capteur selon la revendication 4 ou 5, dans lequel le boîtier comporte une base (12) et un couvercle (14), dans lequel la base a une première cavité annulaire (26) dans laquelle réside le premier joint torique, et le couvercle a une seconde cavité annulaire (28) dans laquelle réside le second joint torique.

7. Dispositif capteur selon la revendication 6, dans lequel au moins l'une parmi les première et seconde cavités annulaires a une forme de section transversale qui s'amincit d'une dimension plus étroite vers une profondeur de la cavité, à une dimension plus large vers un côté ouvert de la cavité pour améliorer une compression du joint torique résidant dans celle-ci, lorsque la base et le couvercle sont accouplés ensemble
et/ou
dans lequel chacune des première et seconde cavités annulaires a une forme de section transversale qui s'amincit d'une dimension plus étroite vers une profondeur de la cavité, à une dimension plus large vers un côté ouvert de la cavité pour améliorer une compression des joints toriques, lorsque la base et le couvercle sont accouplés ensemble.

8. Dispositif capteur selon l'une quelconque des revendications 1 à 7, dans lequel l'au moins un substrat de la sonde de capteur comprend un premier substrat ayant un ou plusieurs conducteurs électriques ou composants électriques.

9. Dispositif capteur selon la revendication 8, dans lequel l'au moins un substrat de la sonde de capteur comprend un second substrat ayant un côté pourvu d'un enrobage d'un médicament ou d'une substance de traitement, le côté du second substrat étant opposé au premier substrat de la sonde de capteur.

10. Dispositif capteur selon la revendication 9, dans lequel le second substrat a une marque ou un autre indice qui identifie le côté pourvu de l'enrobage du médicament ou de la substance de traitement,
et/ou
dans lequel le second substrat a un second côté qui fait face au premier substrat de la sonde de capteur, le second côté du second substrat n'étant pas enrobé.

11. Sonde de capteur (16) configurée pour l'utilisation dans un dispositif capteur (10) selon l'une quelconque des revendications 1 à 10, dans laquelle la sonde de capteur (16) a au moins un substrat (16a) supporté par le boîtier, le substrat ayant la première partie de longueur et la seconde partie de longueur, la première partie de longueur étant externe au boîtier et conçue pour être insérée dans le patient lorsque le boîtier est fixé au patient, la seconde partie de longueur étant dans ou sur le boîtier et ayant une section qui s'étend à travers la structure d'étanchéité, dans laquelle la section de la seconde partie de longueur de l'au moins un substrat qui s'étend à travers la structure d'étanchéité a au moins un bord latéral ayant un motif de bord tortueux qui améliore un effet d'étanchéité de la structure d'étanchéité au niveau de la section de la seconde partie de longueur de l'au moins un substrat.

12. Dispositif capteur selon la revendication 11, dans lequel le motif de bord tortueux sur au moins un bord latéral comprend un motif de vague ayant une pluralité de crêtes ou une pluralité de vallées.

13. Procédé de fabrication d'un dispositif capteur (10), le procédé comprenant :
la fourniture d'un boîtier conçu pour être fixé à un patient ;
l'agencement d'une structure d'étanchéité à l'intérieur du boîtier ; et
le support d'une sonde de capteur (16) ayant au moins un substrat (16a) par le boîtier, le substrat ayant une première partie de longueur et une seconde partie de longueur, la première partie de longueur étant externe au boîtier et conçue pour être insérée dans le patient lorsque le boîtier est fixé au patient, la seconde partie de longueur étant dans ou sur le boîtier et ayant une section qui s'étend à travers la structure d'étanchéité ;
dans lequel la section de la seconde partie de longueur de l'au moins un substrat qui s'étend à travers la structure d'étanchéité a au moins un bord latéral ayant un motif de bord ondulé qui renforce un effet d'étanchéité de la structure d'étanchéité au niveau de la section de la seconde partie de longueur de l'au moins un substrat, en particulier
dans lequel le motif de bord ondulé sur l'au moins un bord latéral comprend un motif de vague ayant une pluralité de crêtes ou une pluralité de vallées.

14. Procédé selon la revendication 13, dans lequel l'agencement d'une structure d'étanchéité à l'intérieur du boîtier comprend :
l'agencement d'un premier joint torique (22) sur une base (12) du boîtier, le premier joint torique ayant une ouverture centrale ;
l'agencement d'une partie de l'au moins un substrat de sorte qu'elle s'étend à travers l'ouverture dans le premier joint torique ; et
l'agencement du second joint torique (24) sur le premier joint torique, l'ouverture centrale du second joint torique étant alignée sur l'ouverture centrale du premier joint torique ; et
l'extension d'une section d'une seconde partie de l'au moins un substrat entre le premier joint torique et le second joint torique.

15. Procédé selon la revendication 13 ou 14, dans lequel :
le boîtier comporte une base (12) et un couvercle (14) conçus pour s'accoupler ensemble ;
l'agencement d'un premier joint torique (22) comprend la mise en place du premier joint torique dans une première cavité annulaire (26) de la base ;
l'agencement du second joint torique (24) comprend la mise en place du second joint torique dans une seconde cavité annulaire (28) du couvercle ;
au moins l'une parmi les première et seconde cavités annulaires a une forme de section transversale qui s'amincit d'une dimension plus étroite vers une profondeur de la cavité, à une dimension plus large vers un côté ouvert de la cavité pour améliorer une compression du joint torique résidant dans celle-ci, lorsque la base et le couvercle sont accouplés ensemble.
